# EUROPÄISCHE PATENTSCHRIFT

(11) **EP 2 026 718 B1**
(45) Veröffentlichungstag und Bekanntmachung des Hinweises auf die Patenterteilung: **15.12.2010**
(21) Anmeldenummer: 07722231.3
(22) Anmeldetag: 17.04.2007
(51) Int. Cl.: A61F 2/38

(54) **KNIE-ENDOPROTHESE**
KNEE ENDOPROSTHESIS
ENDOPROTHÈSE DE GENOU

(30) Priorität: 17.05.2006 DE 102006023378
(43) Veröffentlichungstag der Anmeldung: 25.02.2009
(73) Patentinhaber: Seyfert, Christine, 16866 Kyritz (DE)
(72) Erfinder: Seyfert, Christine, 16866 Kyritz (DE)
(74) Vertreter: Bittner, Thomas L.
(86) Internationale Anmeldenummer: PCT/DE2007/000673
(87) Internationale Veröffentlichungsnummer: WO 2007/131466

(56) Entgegenhaltungen:
- US-A- 5 062 852
- US-A1- 2004 064 191
- US-A1- 2005 107 884
- US-A1- 2006 004 460

## Beschreibung

Die Erfindung betrifft eine Knie-Endoprothese mit einer auf dem Tibiaplateau anordenbaren Prothesenanordnung, wobei die Prothesenanordnung eine mediale und eine laterale Komponente aufweist.

Knie-Endoprothesen der eingangs genannten Art sind aus der Praxis bekannt und existieren in unterschiedlichen Ausgestaltungen und können zusätzlich einen oder zwei Stege aufweisen. Künstliche Kniegelenke in Form von Totalendoprothesen werden Patienten bei erheblichem Verschleiß der Gelenkoberfläche oder kompletter Destruktion des Gelenks implantiert. Die Rekonstruktion einer weitgehend normalen Biomechanik im Kniegelenk ist dabei höchstes Ziel.

Die Standard-Knie-Totalendoprothese wird üblicherweise unter Resektion der Knochenoberfläche und des vorderen Kreuzbands implantiert. Durch die Resektion des vorderen Kreuzbands opfert man außer einem Großteil der Propriozeption auch wichtige biomechanische Elemente wie beispielsweise die Schlussrotation der Tibia bei Streckung. Patienten mit erhaltenem vorderem Kreuzband wünschen deshalb zunehmend Prothesenkornponenten, die eine noch biomechanisch gesunde anatomische Struktur erhalten.

Die derzeitige Forschung bezüglich neuer Knieimplantate ist daher auf den Erhalt des vorderen Kreuzbands fokussiert. Einige wenige Modelle in der Knieendoprothetik, bei denen das vordere Kreuzband erhalten werden kann, sind auf dem Markt.

Das vordere Kreuzband entspringt von der Eminentia der Tibia und verläuft nach lateral zum lateralen Femurkondylus, um dort in der Notch anzusetzen. Der Ursprung des vorderen Kreuzbands umfasst jedoch nicht nur die Eminentia, sondern ist die zentrale ventrale Tibiakante umgreifend breit gefächert.

Herkömmliche künstliche Tibiaplateaus, die das vordere Kreuzband erhalten, weisen einen ventralen Steg auf. Zur Platzierung dieses Stegs, müssen die ventralen Fasern des vorderen Kreuzbandes geopfert werden, damit der Steg ventral des Hauptansates des vorderen Kreuzbands vorbeigeführt werden kann. Eine zu weit reichende knöcherne Resektion gefährdet dabei außer der obligaten Schädigung der ventralen Anteile auch den Rest des vorderen Kreuzbands.

Eine weitere Möglichkeit zum Erhalt des vorderen Kreuzbands besteht in der Implantation von zwei Hemischlitten, die als mediale und laterale Komponenten dienen. Hierbei ist jedoch der Implantationsfehler in Form einer nicht exakt symmetrischen Ausrichtung der ungekoppelten medialen und lateralen Schlitten sehr hoch, so dass diese Methode in jüngerer Zeit kaum noch Anwendung findet.

Das Dokument US 2005/107884 A1 beschreibt ein modulares Knieprothesensystem mit femuraler und tibialer Knieprothese, wobei die mediale und laterale Komponente der tibialen Knieprothese mittels eines Verbindungs- oder Arretierungsmechanismus' verbunden sind, welcher sich über einen Großteil der Ausdehnung von vorne nach hinten erstreckt.

Die Druckschrift US 5 062 852 A zeigt eine tibiale Prothese mit medialen und lateralen Basisplatten und einer Komponente mit einer Aussparung für das hintere Kreuzband. Diese Druckschrift bildet die Basis für den Oberbegriff des Anspruchs 1.

Das Dokument US 2006/004460 A1 offenbart eine Knie-Endoprothese, bei der eine tibiale Komponente mit medialer und lateraler Komponente vorgesehen ist, welche miteinander mittels einer flexiblen Komponente verbunden sind.

Die Druckschrift US 2004/064191 A1 schlägt eine Probeprothese mit einer Sensoranordnung vor, die einen Chirurgen beim Auffinden einer geeigneten, tatsächlich einzusetzenden Prothese unterstützen soll.

Der vorliegenden Erfindung liegt daher die Aufgabe zugrunde, eine Knie-Endoprothese der eingangs genannten Art derart auszugestalten und weiterzubilden, dass ein sicherer Erhalt des vorderen Kreuzbands mit konstruktiv einfachen Mitteln erreicht ist.

Die voranstehende Aufgabe ist durch eine Knie-Endoprothese mit den Merkmalen des Patentanspruchs 1 gelöst. Danach ist die Knie-Endoprothese der eingangs genannten Art derart ausgestaltet und weitergebildet, dass die mediale und die laterale Komponente mittels eines bezüglich des vorderen Kreuzbands dorsal anordnenbaren Verbindungselements koppelbar sind.

In erfindungsgemäßer Weise ist erkannt worden, dass durch die Ausgestaltung eines geeigneten Verbindungselements für die mediale und laterale Komponente die voranstehende Aufgabe auf überraschen einfache Weise gelöst ist. Im Konkreten ist das die mediale und die laterale Komponente koppelnde Verbindungselement bezüglich des vorderen Kreuzbands dorsal anordenbar. Hierdurch wird quasi ein Freiraum für das vordere Kreuzband gebildet, so dass eine Schädigung oder teilweise Resektion des vorderen Kreuzbands nicht erforderlich ist. Das vordere Kreuzband bleibt somit unberührt und die gesamten propriozeptiven Aufgaben sind geschützt. Hierdurch ist der Erhalt eines biomechanisch annähernd natürlichen Gelenks ermöglicht, an welchem nur die zerstörten Oberflächen der Tibia unter Bildung eines geeigneten künstlichen Tibiaplateaus ersetzt werden.

Folglich ist mit der erfindungsgemäßen Knie-Endoprothese eine Knie-Endoprothese angegeben, bei der ein sicherer Erhalt des vorderen Kreuzbands mit konstruktiv einfachen Mitteln erreicht ist.

Im Konkreten könnte das Verbindungselement als Steg ausgebildet sein, der dorsal zwischen dem vorderen und dem hinteren Kreuzband gelagert ist. Somit ist eine Schädigung der Kreuzbänder vermieden.

Hinsichtlich einer besonders sicheren und einfachen Kopplung der Komponenten, die in vorteilhafter Weise wie das mediale und das laterale Tibiaplateau geformt sind, könnten oder könnte die mediale und/oder die laterale Komponente über eine Steckverbindung mit dem Verbindungselement koppelbar sein. Aufgrund der Einfachheit der Kopplung der Komponenten ist die Knie-Endoprothese zur minimal invasiven Operationstechnik geeignet.

In besonders vorteilhafter Weise könnte die Steckverbindung eine konische Steckverbindung sein. Hierdurch ist eine besonders einfache und sichere Steckverbindung bereitgestellt. Die hierdurch erreichte Verbindung entspricht einer Kaltverschweißtechnik.

Hinsichtlich einer besonders guten Verträglichkeit der Knie-Endoprothese im Körper des Patienten könnten oder könnte die mediale Komponente und/oder laterale Komponente und/oder das Verbindungselement ein biomechanisch dem Knochen ähnliches Material aufweisen. Als besonders geeignet hat sich diesbezüglich Tantal oder Trabecular Metal® als Material oder ein dem Trabecular Metal® ähnliches Material gezeigt.

Hinsichtlich einer besonders einfach handhabbaren Knie-Endoprothese könnte die mediale oder die laterale Komponente integral mit dem Verbindungselement ausgebildet sein. Hierdurch ist quasi eine Vormontage einer der beiden Komponenten mit dem Verbindungselement realisiert, so dass während einer Operation nur noch die Verbindung zwischen der verbliebenen Komponente und dem Verbindungselement herzustellen ist. Dies spart Operationszeit. Im Falle dieser Ausgestaltung besteht die Prothesenanordnung für den Bereich der Tibia nicht mehr aus drei sondern nur noch aus zwei modularen Komponenten.

In besonders vorteilhafter Weise könnte die Prothesenanordnung mit dem Verbindungselement vollständig modular aufgebaut sein. Hierdurch ist ein hohes Maß an Anpassbarkeit an unterschiedliche physiologische Gegebenheiten realisiert. Im Konkreten ist aufgrund der Modularität eine hohe Varianz an Größen gegeben, die sich beispielsweise in unterschiedlichen Größen der medialen und lateralen künstlichen Tibiaplateaus bzw. Komponenten und in unterschiedlichen Steglängen bzw. Verbindungselementlängen zeigt. Hierdurch ist in besonderem Maße die Möglichkeit zur minimal invasiven Operationstechnik gegeben, wobei das vordere Kreuzband nicht geschädigt wird.

Bei der erfindungsgemäßen Knie-Endoprothese ist an den herkömmlichen Femurkomponenten keine entscheidende Änderung vorgesehen. Im Hinblick auf eine besonders lang anhaltende und sichere Funktion der Knie-Endoprothese könnte die der Tibia abgewandte Oberseite der Prothesenanordnung mindestens eine Aufnahme für mindestens ein Inlay aufweisen. Ein derartiges Inlay oder derartige Inlays könnten in besonders vorteilhafter Weise vorzugsweise hochvernetzte PE-Inlays sein. Die Inlays dienen einem sicheren Gleiten der Prothesenkomponenten.

Bei einer besonders vorteilhaften Ausgestaltung könnte ein Inlay medial und ein weiteres Inlay lateral in der Aufnahme oder in den Aufnahmen angeordnet sein. Damit könnte jedem Femurkondylus ein Inlay zugeordnet sein. Hierzu könnte in weiter besonders vorteilhafter und einfacher Weise sowohl die mediale als auch die laterale Komponente jeweils eine Aufnahme aufweisen.

In weiter vorteilhafter Weise könnte das Inlay oder könnten die Inlays in einem Lager gleitend angeordnet sein. Zur Gewährleistung einer individuellen Anpassung der Inlays an die physiologischen Gegebenheiten könnte das Inlay oder könnten die Inlays in unterschiedlichen Höhen und/oder Größen verfügbar sein.

Die tibialen Komponenten - medial, lateral und Steg - sowie die femuralen Komponenten können in verschiedenen, kombinierbaren Größen vorgehalten werden.

In besonderen Fällen könnte das Verbindungselement eine zusätzliche ventral des vorderen Kreuzbands geführte vorzugsweise als Steg ausgebildete Komponente aufweisen.

Die erfindungsgemäße Knie-Endoprothese ist vorzugsweise für die zementfreie Implantationstechnik vorgesehen. An den herkömmlichen Femurkomponenten sind keine entscheidenden Änderungen erforderlich. Die Unterfläche der Prothesenanordnung genügt dabei den gleichen Ansprüchen wie herkömmliche Prothesenanordnungen oder Plateaus für die zementfreie Implantation.

Mit der erfindungsgemäßen Knie-Endoprothese ist eine neue Tibiakomponente einer Knie-Endoprothese unter Erhalt der Kreuzbänder bereitgestellt. Das Verbindungselement der medialen und lateralen Komponenten führt dorsal des vorderen Kreuzbands entlang und nicht ventral. Dabei handelt es sich um ein modulares System, welches durch konische Steckverbindungen kalt verschweißt werden kann. Es ist zur minimal invasiven Operationstechnik geeignet und aufgrund der Modularität ist die Größenanpassung optimal gewährleistet. Dabei ist eine separate Größenwahl für die medialen und lateralen Komponenten sowie für das Verbindungselement sowie die Inlayhöhe möglich.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung eines Ausführungsbeispiels der erfindungsgemäßen Knie-Endoprothese anhand der Zeichnung zu verweisen. In Verbindung mit der Erläuterung des bevorzugten Ausführungsbeispiels der erfindungsgemäßen Knie-Endoprothese anhand der Zeichnung werden auch im Allgemeinen bevorzugte Ausgestaltungen und Weiterbildungen der Lehre erläutert. In der Zeichnung zeigen
- Fig. 1: in einer schematischen Draufsicht ein normales Tibiaplateau und
- Fig. 2: in einer schematischen Draufsicht ein Ausführungsbeispiel einer auf dem Tibiaplateau angeordneten Prothesenanordnung einer erfindungs- gemäßen Knie-Endoprothese.

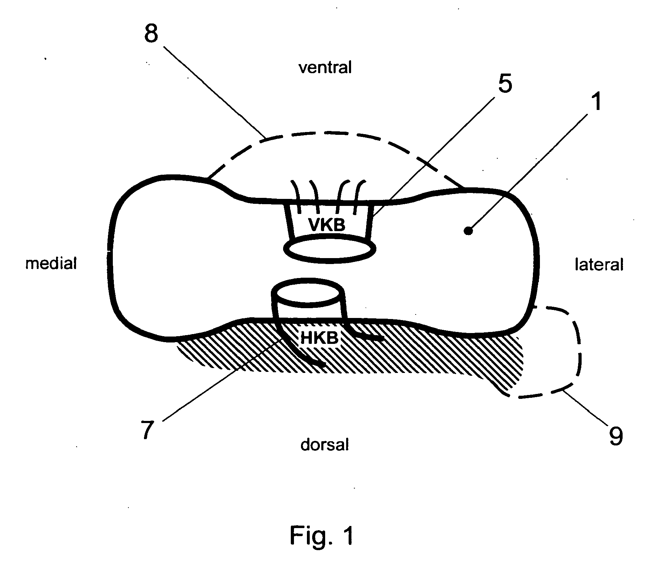

Fig. 1 zeigt in einer schematischen Draufsicht das Plateau 1 der Tibia, wobei das vordere Kreuzband 5 und das hintere Kreuzband 7 am vorderen bzw. hinteren Bereich der Tibia dargestellt sind. Des Weiteren sind in gestrichelten Linien die Tuberositas tibiae 8 sowie das Fibulaköpfchen 9 dargestellt.

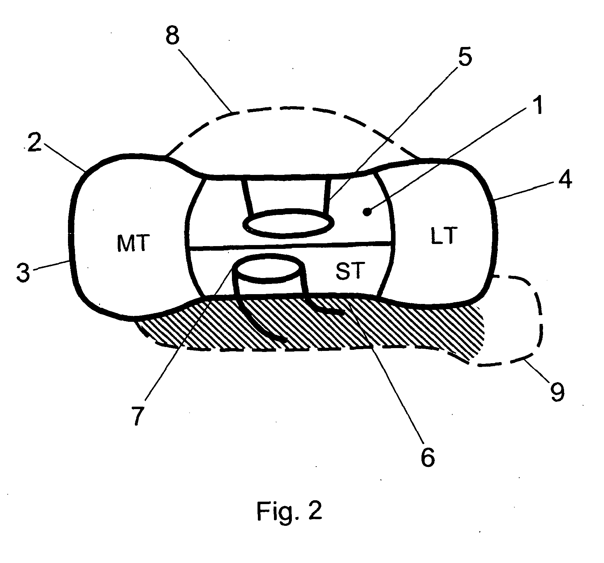

Fig. 2 zeigt in einer schematischen Draufsicht das Tibiaplateau 1 mit einer darauf angeordneten Prothesenanordnung 2 eines Ausführungsbeispiels einer erfindungsgemäßen Knie-Endoprothese. Die Prothesenanordnung 2 weist eine mediale Komponente 3 und eine laterale Komponente 4 auf. Im Hinblick auf einen sicheren Erhalt des vorderen Kreuzbands 5 sind die mediale Komponente 3 und die laterale Komponente 4 mittels eines bezüglich des vorderen Kreuzbands 5 dorsal anordenbaren Verbindungselements 6 koppelbar. Die mediale Komponente 3, die laterale Komponente 4 sowie das Verbindungselement 6 sind in dicken Linien gezeichnet.

Unter der Prothesenanordnung 2 erkennt man die Tuberositas tibiae 8 sowie das Fibulaköpfchen 9. Das vordere Kreuzband 5 liegt zwischen der medialen Komponente 3 und der lateralen Komponente 4. Das hintere Kreuzband 7 ragt über das Verbindungselement 6 hinaus.

Das Verbindungselement 6 ist als Steg ausgebildet und koppelt die mediale Komponente 3 und die laterale Komponente 4 über konische Steckverbindungen, die der Übersichtlichkeit halber hier nicht gezeigt sind. Die Steckverbindungen sind in ihrer Dimensionierung von der Art des Materials der Komponenten 3 und 4 und des Verbindungselements 6 und der Dicke des Implantats insgesamt abhängig.

Hinsichtlich weiterer vorteilhafter Ausgestaltungen der erfindungsgemäßen Knie-Endoprothese wird zur Vermeidung von Wiederholungen auf den allgemeinen Teil der Beschreibung sowie auf die beigefügten Patentansprüche verwiesen.

Schließlich sei ausdrücklich darauf hingewiesen, dass das voranstehend beschriebene Ausführungsbeispiel der erfindungsgemäßen Knie-Endoprothese lediglich zur Erörterung der beanspruchten Lehre dient, diese jedoch nicht auf das Ausführungsbeispiel einschränkt.

## Patentansprüche

1. Knie-Endoprothese mit einer auf dem Tibiaplateau (1) anordenbaren Prothesenanordnung (2), wobei die Prothesenanordnung (2) eine mediale (3) und eine laterale Komponente (4) aufweist,
**dadurch gekennzeichnet, dass** die mediale (3) und die laterale Komponente (4) mittels eines bezüglich des vorderen Kreuzbands (5) dorsal anordenbaren Verbindungselements (6) koppelbar sind.

2. Knie-Endoprothese nach Anspruch 1, **dadurch gekennzeichnet, dass** das Verbindungselement (6) als Steg ausgebildet ist.

3. Knie-Endoprothese nach Anspruch 1 oder 2, **dadurch gekennzeichnet, dass** die mediale und/oder die laterale Komponente (3, 4) über eine Steckverbindung mit dem Verbindungselement (6) koppelbar sind oder ist.

4. Knie-Endoprothese nach Anspruch 3, **dadurch gekennzeichnet, dass** die Steckverbindung eine konische Steckverbindung ist.

5. Knie-Endoprothese nach einem der Ansprüche 1 bis 4, **dadurch gekennzeichnet, dass** die mediale Komponente (3) und/oder die laterale Komponente (4) und/oder das Verbindungselement (6) ein biomechanisch dem Knochen ähnliches Material aufweist oder aufweisen.

6. Knie-Endoprothese nach Anspruch 5, **dadurch gekennzeichnet, dass** das Material Tantal oder Trabecular Metal® oder ein dem Trabecular Metal® ähnliches Material ist.

7. Knie-Endoprothese nach einem der Ansprüche 1 bis 6, **dadurch gekennzeichnet, dass** die mediale (3) oder die laterale Komponente (4) integral mit dem Verbindungselement (6) ausgebildet ist.

8. Knie-Endoprothese nach einem der Ansprüche 1 bis 7, **dadurch gekennzeichnet, dass** die Prothesenanordnung (2) mit dem Verbindungselement (6) modular aufgebaut ist.

9. Knie-Endoprothese nach einem der Ansprüche 1 bis 8, **dadurch gekennzeichnet, dass** die der Tibia abgewandte Oberseite der Prothesenanordnung (2) mindestens eine Aufnahme für mindestens ein Inlay aufweist.

10. Knie-Endoprothese nach Anspruch 9, **dadurch gekennzeichnet, dass** das mindestens eine Inlay ein vorzugsweise hochvernetztes PE-Inlay ist.

11. Knie-Endoprothese nach Anspruch 9 oder 10, **dadurch gekennzeichnet, dass** ein Inlay medial und ein weiteres Inlay lateral in der Aufnahme oder in den Aufnahmen angeordnet sind.

12. Knie-Endoprothese nach einem der Ansprüche 9 bis 11, **dadurch gekennzeichnet, dass** sowohl die mediale (3) als auch die laterale Komponente (4) jeweils eine Aufnahme aufweisen.

13. Knie-Endoprothese nach einem der Ansprüche 9 bis 12, **dadurch gekennzeichnet, dass** das Inlay oder die Inlays in einem Lager gleitend angeordnet ist oder sind.

14. Knie-Endoprothese nach einem der Ansprüche 1 bis 13, **dadurch gekennzeichnet, dass** das Verbindungselement (6) eine zusätzliche ventral des vorderen Kreuzbands (5) geführte vorzugsweise als Steg ausgebildete Komponente aufweist.

## Claims

1. A knee endoprosthesis comprising a prosthesis arrangement (2) that can be placed on the tibial plateau (1), wherein the prosthesis arrangement (2) has a medial component (3) and a lateral component (4), **characterized in that** the medial component (3) and the lateral component (4) can be coupled by means of a connecting element (6) which can be placed dorsally in relation to the anterior cruciate ligament (5).

2. The knee endoprosthesis according to claim 1, **characterized in that** the connecting element (6) is configured as a web.

3. The knee endoprosthesis according to claim 1 or 2, **characterized in that** the medial and/or lateral component (3, 4) can be coupled via a plug-type connection to the connecting element (6).

4. The knee endoprosthesis according to claim 3, **characterized in that** the plug-type connection is a conical plug-type connection.

5. The knee endoprosthesis according to any one of the claims 1 to 4, **characterized in that** the medial component (3) and/or the lateral component (4) and/or the connecting element (6) comprises a material which is biomechanically similar to bone.

6. The knee endoprosthesis according to claim 5, **characterized in that** the material is tantalum or Trabecular Metal^{®} or a material similar to Trabecular Metal^{®}.

7. The knee endoprosthesis according to any one of the claims 1 to 6, **characterized in that** the medial component (3) or the lateral component (4) is configured to be integral with the connecting element (6).

8. The knee endoprosthesis according to any one of the claims 1 to 7, **characterized in that** the prosthesis arrangement (2) is constructed in a modular manner, together with the connecting element (6).

9. The knee endoprosthesis according to any one of the claims 1 to 8, **characterized in that** the upper side of the prosthesis arrangement (2) facing away from the tibia has at least one receptacle for at least one inlay.

10. The knee endoprosthesis according to claim 9, **characterized in that** the at least one inlay is preferably a highly cross-linked PE inlay.

11. The knee endoprosthesis according to claim 9 or 10, **characterized in that** one inlay is arranged medially and a further inlay is placed laterally, in the receptacle or receptacles.

12. The knee endoprosthesis according to any one of the claims 9 to 11, **characterized in that** both the medial component (3) and the lateral component (4) each comprise a receptacle.

13. The knee endoprosthesis according to any one of the claims 9 to 12, **characterized in that** the inlay or inlays is or are slideably placed in a bearing.

14. The knee endoprosthesis according to any one of the claims 1 to 13, **characterized in that** the connecting element (6) has an additional component, preferably configured as a web and arranged ventrally in relation to the anterior cruciate ligament (5).

## Revendications

1. Endoprothèse du genou avec un ensemble prothétique (2) pouvant être disposé sur le plateau tibial (1), étant donné que l'ensemble prothétique (2) présente une composante médiale (3) et une composante latérale (4),
**caractérisée en ce que** la composante médiale (3) et la composante latérale (4) peuvent être accouplées au moyen d'un élément de liaison (6) pouvant être disposé dorsalement par rapport au ligament croisé avant (5).

2. Endoprothèse du genou selon la revendication 1, **caractérisée en ce que** l'élément de liaison (6) est formé comme entretoise.

3. Endoprothèse du genou selon la revendication 1 ou 2, **caractérisée en ce que** la composante médiale et/ou latérale (3, 4) peut être accouplé(e) via connexion à fiche à l'élément de liaison (6).

4. Endoprothèse du genou selon la revendication 3, **caractérisée en ce que** la connexion à fiche est une connexion à fiche conique.

5. Endoprothèse du genou selon l'une quelconque des revendications 1 à 4, **caractérisée en ce que** la composante médiale (3) et/ou la composante latérale (4) et/ou l'élément de liaison (6) présente(nt) un matériau biomécanique similaire à l'os.

6. Endoprothèse du genou selon la revendication 5, **caractérisée en ce que** le matériau est le Tantal ou Trabecular Metal^{®} ou un matériau similaire au Trabecular Metal^{®}.

7. Endoprothèse du genou selon l'une quelconque des revendications 1 à 6, **caractérisée en ce que** la composante médiale (3) ou la composante latérale (4) est intégrale avec l'élément de liaison (6).

8. Endoprothèse du genou selon l'une quelconque des revendications 1 à 7, **caractérisée en ce que** l'ensemble prothétique (2) est modulaire avec l'élément de liaison (6).

9. Endoprothèse du genou selon l'une quelconque des revendications 1 à 8, **caractérisée en ce que** la face supérieure orientée vers le tibia de l'ensemble prothétique (2) présente au moins un logement pour au moins un inlay.

10. Endoprothèse du genou selon la revendication 9, **caractérisée en ce que** le au moins un inlay est de préférence un inlay en PE hautement réticulé.

11. Endoprothèse du genou selon la revendication 9 ou 10, **caractérisée en ce qu'**un inlay est disposé médialement et un autre inlay disposé latéralement dans le ou les logements.

12. Endoprothèse du genou selon l'une quelconque des revendications 9 à 11, **caractérisée en ce qu'**aussi bien la composante médiale (3) que la composante latérale (4) présentent chacune un logement.

13. Endoprothèse du genou selon l'une quelconque des revendications 9 à 12, **caractérisée en ce que** l'inlay ou les inlays est (sont) disposé(s) de manière coulissante dans un palier.

14. Endoprothèse du genou selon l'une quelconque des revendications 1 à 13, **caractérisée en ce que** l'élément de liaison (6) présente une composante supplémentaire guidée ventralement par rapport au ligament croisé avant (5), de préférence formée comme entretoise.
